# EUROPEAN PATENT SPECIFICATION

(11) **EP 2 073 849 B1**
(45) Date of publication and mention of the grant of the patent: **10.11.2010**
(21) Application number: 07867164.1
(22) Date of filing: 28.09.2007
(51) Int. Cl.: A61K 47/48, A61K 48/00, C12N 15/861

(54) **METHODS AND COMPOSITIONS FOR GENE THERAPY**
VERFAHREN UND ZUSAMMENSETZUNGEN FÜR GENTHERAPIE
PROCÉDÉS ET COMPOSITIONS POUR UNE THÉRAPIE GÉNIQUE

(30) Priority: 29.09.2006 US 848614 P
(43) Date of publication of application: 01.07.2009
(73) Proprietor: CANJI, Inc., Palo Alto, California 94304 (US)
(72) Inventor: LAFACE, Drake M., Half Moon Bay, CA 94019 (US); TSAI, Van T., San Diego, CA 92130 (US)
(74) Representative: van Wezenbeek, Petrus M.G.F.
(86) International application number: PCT/US2007/020931
(87) International publication number: WO 2008/060356

(56) References cited:
- WO-A-00/11202
- WO-A-2007/062207
- CROYLE M A ET AL: "PEGylated helper-dependent adenoviral vectors: highly efficient vectors with an enhanced safety profile." GENE THERAPY APR 2005, vol. 12, no. 7, April 2005 (2005-04), pages 579-587, XP002479207 ISSN: 0969-7128
- DE GEEST BART ET AL: "Elimination of innate immune responses and liver inflammation by PEGylation of adenoviral vectors and methylprednisolone." HUMAN GENE THERAPY DEC 2005, vol. 16, no. 12, December 2005 (2005-12), pages 1439-1451, XP002479208 ISSN: 1043-0342
- MOK H ET AL: "Evaluation of polyethylene glycol modification of first-generation and helper-dependent adenoviral vectors to reduce innate immune responses" MOLECULAR THERAPY, ACADEMIC PRESS, SAN DIEGO, CA,, US, vol. 11, no. 1, January 2005 (2005-01), pages 66-79, XP004672528 ISSN: 1525-0016
- LEE SEOJU ET AL: "Investigations of PEGylated recombinant adenovirus, using fluorescein-labeled polyethylene glycol" HUMAN GENE THERAPY, MARY ANN LIEBERT, NEW YORK ,NY, US, vol. 18, no. 3, March 2007 (2007-03), pages 286-300, XP002446936 ISSN: 1043-0342

## Description

### REFERENCE TO CROSS RELATED APPLICATIONS

This application claims the benefit of priority under 35 USC 119(e) of provisional patent application U.S.S.N.: 60/848,614 filed September 29, 2006, the disclosure of which is hereby incorporated by reference in its entirety.

### FIELD OF THE INVENTION

The present invention relates to improved methods for gene therapy, particularly gene therapy using PEGylated adenovirus.

### BACKGROUND OF THE INVENTION

Dose dependent adverse effects have been associated with systemic administration recombinant adenoviruses for gene therapy. As with other types of therapies, the desire for adenoviral based gene therapy is to mitigate adverse effects while maximizing therapeutic efficacy.

### SUMMARY OF THE INVENTION

The present invention provides a method of mitigating adverse effects associated with systemic administration of a recombinant adenovirus for gene therapy, said method comprising administering a PEGylated recombinant adenovirus virion to a subject, wherein the degree of PEGylation of the adenovirus virion is between about 400 PEG molecules per virion and about 1250 PEG molecules per virion..

The present invention provides a method of mitigating adverse effects associated with systemic administration of a recombinant adenovirus for gene therapy, said method comprising administering a PEGylated recombinant adenovirus virion to a subject, wherein the degree of PEGylation of the adenovirus virion is between about 400 PEG molecules per virion and about 1250 PEG molecules per virion, such as, for example, between about 600 PEG molecules per virion and about 1000 PEG molecules per virion .

The present invention also provides a method of mitigating adverse effects associated with systemic administration of a recombinant adenovirus for gene therapy, said method comprising administering a PEGylated recombinant adenovirus virion to a subject, wherein the degree of PEGylation of the adenovirus virion is about 1250 PEG molecules per virion.

In one embodiment the invention provides a method of mitigating cardiovascular effects associated with systemic administration of a recombinant adenovirus for gene therapy, said method comprising administering a PEGylated adenovirus virion to a subject wherein the degree of PEGylation of the adenovirus virion is between about 400 PEG molecules per virion and about 1250 PEG molecules per virion, such as, for example, between about 600 PEG molecules per virion and about 1000 PEG molecules per virion.

In another embodiment the invention provides a method of mitigating cardiovascular effects associated with systemic administration of a recombinant adenovirus for gene therapy, said method comprising administering a PEGylated adenovirus virion to a subject wherein the degree of PEGylation of the adenovirus virion is about 1250 PEG molecules per virion.

In another embodiment the invention provides a method of mitigating coagulopathy associated with systemic administration of a recombinant adenovirus for gene therapy, said method comprising administering a PEGylated adenovirus virion to a subject wherein the degree of PEGylation of the adenovirus virion is between about 400 PEG molecules per virion and about 1250 PEG molecules per virion, such as, for example, between about 600 PEG molecules per virion and about 1000 PEG molecules per virion.

In another embodiment the invention provides a method of mitigating coagulopathy associated with systemic administration of a recombinant adenovirus for gene therapy, said method comprising administering a PEGylated adenovirus virion to a subject wherein the degree of PEGylation of the adenovirus virion is about 1250 PEG molecules per virion.

In another embodiment the invention provides a method of mitigating anaphylactoid responses associated with systemic administration of a recombinant adenovirus vector for gene therapy, said method comprising administering a PEGylated adenovirus virion to a subject wherein the degree of PEGylation of the adenovirus virion is between about 400 PEG molecules per virion and about 1250 PEG molecules per virion, such as, for example, between about 600 PEG molecules per virion and about 1000 PEG molecules per virion .

In another embodiment the invention provides a method of mitigating anaphylactoid responses associated with systemic administration of a recombinant adenovirus vector for gene therapy, said method comprising administering a PEGylated adenovirus virion to a subject wherein the degree of PEGylation of the adenovirus virion is about 1250 PEG molecules per virion.

In yet another embodiment the invention provides a method of mitigating adverse effects associated with systemic administration of a recombinant adenovirusl for gene therapy, said method comprising administering a PEGylated adenovirus virion to a subject wherein the degree of PEGylation of the adenovirus virion is between about 400 PEG molecules per virion and about 1250 PEG molecules per virion, such as, for example, between about 600 PEG molecules per virion and about 1000 PEG molecules per virion and wherein the transduction efficiency of the PEGylated recombinant adenovirus is equal to or greater then the transduction efficiency of a non-PEGylated recombinant adenovirus..

### DETAILED DESCRIPTION OF THE INVENTION

The present invention provides PEGylated recombinant adenovirus with a degree of PEGylation that mitigates adverse effects associated with systemic administration of a recombinant adenovirus. Without being bound by theory, it is believed that the PEGylation of adenoviral capsid proteins inhibits the binding of cell receptors capable of inducing the release of immediate inflammatory mediators such as histamines and cytokines by stearic hindrance of the adenoviral proteins with the respective receptor(s). It is also believed that the PEGylation may inhibit interactions with blood components capable of inducing disseminated coagulopathies induced by recombinant adenoviral vectors in a subject.

The present invention provides a method of mitigating adverse effects associated with systemic administration of a recombinant adenovirus for gene therapy, said method comprising administering a PEGylated recombinant adenovirus virion to a subject, wherein the degree of PEGylation of the adenovirus virion is between about 400 PEG molecules per virion and about 1250 PEG molecules per virion..

In one embodiment the present invention provides a method of mitigating adverse effects associated with systemic administration of a recombinant adenovirus for gene therapy, said method comprising administering a PEGylated recombinant adenovirus virion to a subject, wherein the degree of PEGylation of the adenovirus virion is between about 400 PEG molecules per virion and about 1250 PEG molecules per virion such as, for example, between about 600 PEG molecules per virion and about 1000 PEG molecules per virion .

In another embodiment the present invention provides a method of mitigating adverse effects associated with systemic administration of a recombinant adenovirus for gene therapy, said method comprising administering a PEGylated recombinant adenovirus virion to a subject, wherein the degree of PEGylation of the adenovirus virion is about 1250 PEG molecules per virion.

In one embodiment the invention provides a method of mitigating cardiovascular effects associated with systemic administration of a recombinant adenovirus for gene therapy, said method comprising administering a PEGylated recombinant adenovirus virion to a subject, wherein the degree of PEGylation of the adenovirus virion is between about 400 PEG molecules per virion and about 1250 PEG molecules per virion such as, for example, between about 600 PEG molecules per virion and about 1000 PEG molecules per virion.

In one embodiment the invention provides a method of mitigating cardiovascular effects associated with systemic administration of a recombinant adenovirus for gene therapy, said method comprising administering a PEGylated recombinant adenovirus virion to a subject, wherein the degree of PEGylation of the adenovirus virion is about 1250 PEG molecules per virion.

In one embodiment the invention provides a method of mitigating coagulopaty associated with systemic administration of a recombinant adenovirus for gene therapy, said method comprising administering a PEGylated recombinant adenovirus virion to a subject, wherein the degree of PEGylation of the adenovirus virion is between about 400 PEG molecules per virion and about 1250 PEG molecules per virion such as, for example, between about 600 PEG molecules per virion and about 1000 PEG molecules per virion.

In yet another embodiment the invention provides a method of mitigating coagulopaty associated with systemic administration of a recombinant adenovirus for gene therapy, said method comprising administering a PEGylated recombinant adenovirus virion to a subject, wherein the degree of PEGylation of the adenovirus virion is about 1250 PEG molecules per virion.

In another embodiment the invention provides a method of mitigating anaphylactoid responses associated with systemic administration of a recombinant adenoviral for gene therapy including, but not limited subjects predisposed to inflammatory conditions and/or having inflammatory conditions, said method comprising administering a PEGylated recombinant adenovirus virion to a subject, wherein the degree of PEGylation of the adenovirus virion is between about 400 PEG molecules per virion and about 1250 PEG molecules per virion such as, for example, between about 600 PEG molecules per virion and about 1000 PEG molecules per virion.

In another embodiment the invention provides a method of mitigating anaphylactoid responses associated with systemic administration of a recombinant adenoviral for gene therapy including, but not limited subjects predisposed to inflammatory conditions and/or having inflammatory conditions, said method comprising administering a PEGylated recombinant adenovirus virion to a subject, wherein the degree of PEGylation of the adenovirus virion is about 1250 PEG molecules per virion.

In yet another embodiment the invention provides a method of mitigating adverse effects associated with systemic administration of a recombinant adenovirus for gene therapy, said method comprising administering a PEGylated adenovirus virion to a subject wherein the degree of PEGylation of the adenovirus virion is between about 400 PEG molecules per virion and about 1250 PEG molecules per virion, such as between about 600 PEG molecules per virion and about 1000 PEG molecules per virion and wherein a transduction efficiency of the PEGylated recombinant adenovirus is equal to or greater than a transduction efficiency of a non-PEGylated recombinant adenovirus. By way of example, and not limitation, transduction efficiency can be higher than non-PEGylated recombinant adenovirus when neutralizing antibodies are present in the serum.

Generally, administration of greater than about 1250 PEG molecules per virion and up to about 1600 PEG molecules per virion is associated with lower transduction efficiency but higher mitigation of adverse effects associated with systemic administration of a recombinant adenovirus for gene therapy. Examples of conditions in a subject in which systemic administration of PEGylated recombinant adenovirus (e.g., a PEGylated replication deficient recombinant adenovirus comprising a polynucleotide sequence encoding a therapeutic protein) can be useful include but are not limited to, chronic inflamatory conditions. Examples of such conditions include but are not limited to COPD, rheumatoid arthritis and cancer.

Methods of measuring transduction efficiency are known in the art.

This invention also provides compositions comprising the PEGylated adenovirus.

### General

In accordance with the present invention there may be employed conventional molecular biology, microbiology, and recombinant DNA techniques within the skill of the art. Such techniques are explained in the literature. See, *e.g*., Sambrook, Fritsch & Maniatis, Molecular Cloning: A Laboratory Manual, Second Edition (1989) Cold Spring Harbor Laboratory Press, Cold Spring Harbor, New York (herein "Sambrook, *et al*., 1989"); DNA Cloning A Practical Approach, Volumes I and II (D.N. Glover ed. 1985); Oligonucleotide Synthesis (M.J. Gait ed. 1984); Nucleic Acid Hybridization (B.D. Hames & S.J. Higgins eds. (1985)); Transcription And Translation (B.D. Hames & S.J. Higgins, eds. (1984)); Animal Cell Culture (R.I. Freshney, ed. (1986)); Immobilized Cells And Enzymes (IRL Press, (1986)); B. Perbal, A Practical Guide To Molecular Cloning (1984); F.M. Ausubel, et al. (eds.), Current Protocols in Molecular Biology, John Wiley & Sons, Inc. (1994).

### PEGylalion of Adenoviruses

The adenovirus virion may be PEGylated by any method know in the art. PEG modification is a well-established technique for the modification of therapeutic peptides and proteins. A primary advantage of PEGylation for proteins and peptides includes a reduction in antigenicity and immunogenicity. Preparation of PEG-protein conjugates requires, in general, activation of hydroxyl groups of PEG with a suitable reagent that can be fully substituted by nucleophilic groups (mainly lysine ε-amino groups) in the protein during the coupling reaction (O'Riordan et al., Hum. Gene Ther. 10:1349-1358 (1999)). A wide variety of methods have been developed to produce activated PEG linkers (Francis et al., J. Drug Target. 3:321-340 (1996)).

Likewise, many methods for conjugation of various polyethylene glycols to the capsid protein of adenoviruses are available ((O'Riordan et al., Hum. Gene Ther. 10:1349-1358 (1999)). Croyle et al. (Hum. Gene Ther. 11:1713-1722 (2000)) describes three conjugation methods with shortened reaction times that sufficiently modify the viral capsid and the physical stability of the adenovirus under extreme storage conditions. Other methods are available in the art. By way of example, and not limitation, the polymer can be conjugated covalently, (see, e.g, U.S.Patent Nos.: 5,711,944 and 5,951,974) or non-covalently (see, e.g., WO2005/012407)

A variety of activated polyethylene glycol linkers is available for the PEGylation of proteins and rAds. PEG-SPA (SPA: succinimidyl ester of PEG propionic acid), which is polyethylene glycol activated with N-hydroxy succinimide (NHS) at one end and capped with a methoxy group at the other, may be selected to pegylate the adenovirus.

PEG-SPA is also available with a fluorescein moiety on the PEG at the opposite end from the NHS ester. The advantage of this fluorescein-labeled PEG linker (here abbreviated as fluoro-PEG-SPA), is that it has the same reactivity as the PEG-SPA. In a preferred embodiment PEG-SPA linker with a PEG molecular weight of 5kDA is used

Other PEG linkers may also be used. For example, tresyl-MPEG (TM-PEG) has been used to successfully pegylate adenoviruses ((O'Riordan et al., Hum. Gene Ther. 10:1349-1358 (1999); Sigma Chemical (St. Louis, MO); Shearwater Polymers (Huntsville, AL); PolyMASC Pharmaceuticals (London, UK)). Other commercially available linkers include succinimidyl succinate MPEG (SS-PEG), polyphthalamide (PPA) and cyanuric chloride MPEG (CC-PEG) (Sigma Chemical Co. (St. Louis, MO).

By way of example, and not limitation, generally between about a 1% w/v PEG to about a 4% w/v PEG will generate between about 400 PEG molecules per virion and about 1250 PEG molecules per virion. Also by way of example, and not limitation, generally between about a 5% w/v PEG to about a 8% w/v PEG will generate greater than about 400 PEG molecules per virion and about 1600 PEG molecules per virion. (See e.g., WO/2007/062207)

### Peglyation of Adenovius

The adenovirus may be Peglyated by any method known in the art. By way of example, and not limitation, the polymer can be conjugated covalently, (see, e.g, U.S.Patent Nos.: 5,711,944 and 5,951,974) or non-covalently (see, e.g., WO2005/012407). (see U.S.S.N.: 60/739,739 filed November 23, 2006, herein incorporated by reference in its entirety). See also U.S.S.N.: 60/739,739, filed November 23, 2006, herein incorporated by reference in its entirety.

### Methods for Determining the Degree of PEGylation

Any method known in the art can also used to determine the relative degree of PEGylation of the virion. In a preferred embodiment the method used to determine the average degree of peglyation of the adenovirus virion utilizes analytical ultracentifugation (AUC) on CsCl gradients (see U.S.S.N.: 60/739,739 filed November 23, 2006, now International Publication No.: WO2007/062207, herein incorporated by reference in its entirety). Other methods which are known in the art can also used to determine the relative degree of PEGylation of the polymer-particle conjugate preparation to be used as a standard. For example, the prior art method of using a biotin-labeled PEG linker that had its DP determined by ELISA analysis of the biotin-labeled PEGylated rAd with avidin-horseradish peroxidase (O'Riordan et al., Hum. Gene Ther. 10:1349-1358 (1999)) can be used as a standard. Alternately, the PEGylated virus preparation can be treated with fluorescamine to quantify the loss of lysine groups relative to unPEGylated controls (Croyle et al., Hum. Gene Ther. 11:1713-1722) for determination of the average degree of PEGylation of the standard preparation.

### Recombinant Adenoviruses (rAd)

Recombinant Adenoviruses (rAd) are widely used to deliver genes into cells for vaccines or gene therapy (Alemany et al., J. Gen. Virol. 81(11):2605-2609 (2000); Vorberger & Hunt, Oncologist 7(1):46-59 (2002); Mizuguchi & Hayakawa, Hum. Gene Ther. 15(11):1034-1044 (2004); Basak et al., Viral Immunol. 172:182-96 (2004). The term "recombinant" refers to a genome which has been modified through conventional recombinant DNA techniques.

The term "adenovirus" is synonymous with the term "adenoviral vector" and refers to viruses of the genus adenoviridae. The term "recombinant adenovirus" is synonymous with the term "recombinant adenoviral vector" and refers to viruses of the genus adenoviridiae capable of infecting a cell, whose viral genomes have been modified through conventional recombinant DNA techniques. The term recombinant adenovirus also includes chimeric (or even multimeric) vectors, *i.e*. vectors constructed using complementary coding sequences from more than one viral subtype.

The term adenoviridae refers collectively to animal adenoviruses of the genus mastadenovirus including but not limited to human, bovine, ovine, equine, canine, porcine, murine and simian adenovirus subgenera. In particular, human adenoviruses include the A-F subgenera as well as the individual serotypes thereof. For example, any of adenovirus types 1, 2, 3, 4, 4a, 5, 6, 7, 7a, 7d, 8, 9, 10, 11 (Ad11A and Ad11P), 12, 13, 14, 15, 16, 17, 18, 19, 20, 21, 22, 23, 24, 25, 26, 27, 28, 29, 30, 31, 32, 33, 34, 34a, 35, 35p, 36, 37, 38, 39, 40, 41, 42, 43, 44, 45, 46, 47, 48, and 91 may be produced in a cell culture of the invention. In the preferred practice of the invention, the adenovirus is or is derived from the human adenovirus serotypes 2 or 5.

The recombinant adenovirus is a recombinant adenovirus or a recombinant adenoviral vector, which comprises a mutated genome; for example the mutated genome may be lacking a segment or may include one or more additional, heterologous gene. In one embodiment, the recombinant adenoviral vector is the adenoviral vector delivery system which has a deletion of the protein IX gene (see International Patent Application WO 95/11984, which is herein incorporated by reference).

In another embodiment, the adenovirus is a selectively replicating recombinant adenovirus or a conditionally replicating adenovirus, *i.e*., an adenovirus that is attenuated in normal cells while maintaining virus replication in tumor cells, see, *e.g*., Kirn, D. et al., Nat. Med. 7:781-787 (2001); Alemany, R. et al. Nature Biotechnology 18: 723-727 (2000); Ramachandra, M. et al., Replicating Adenoviral Vectors for Cancer Therapy in Pharmaceutical Delivery Systems, Marcel Dekker Inc., New York, pp. 321-343 (2003).

In one embodiment of the invention, the selectively replicating recombinant adenovirus or the adenoviral vector is such as those described in published international application numbers, WO 00/22136 and WO 00/22137; Ramachandra, M. et al., Nature Biotechnol. 19: 1035-1041 (2001); Howe et al., Mol. Ther. 2(5):485-95 (2000); and Demers, G. et al. Cancer Research 63: 4003-4008 (2003).

A selectively replicating recombinant adenovirus may also be described as, but not limited to, an "oncolytic adenovirus", an "oncolytic replicating adenovirus", a "replicating adenoviral vector", a "conditionally replicating adenoviral vector" or a "CRAV". In another embodiment of the invention, PEGylation may be used to mitigate adverse responses, as described above, induced by other types of viral vectors. Examples include, but are not limited to retroviral vectors, adeno-associated viral vectors, leniviral vectors and SV40 viral vectors.,

In one embodiment of the recombinant adenovirus of the invention comprise a heterologous nucleotide sequence such as but not limited to a moiety, peptide, polypeptide or protein possessing a desired biological property or activity.

In certain embodiments, the heterologous nucleotide sequence encodes a biological response modifier such as a cytokine, cytokine receptor, hormone, growth factor or growth factor receptor. Non-limiting examples of such biological response modifiers include interferon (IFN)-alpha, IFN-beta, IFN gamma, interleukin (IL-1), IL-2, IL-3, IL-4, IL-5, IL-6, IL-7, IL-9, IL-10, IL-12, IL-15, IL-18, IL-23, erythropoietin (EPO), basic fibroblast growth factor (bFGF), acidic fibroblast growth factor (aFGF), vascular endothelial growth factor (VEGF), platelet derived growth factor (PDGF), epidermal growth factor (EGF), thymic stromal lymphopoietin (TSLP), GM-CSF, TNFR and TNFR ligand superfamily members including TNFRSF 18 and TNFSF18. In a preferred embodiment the nucleotide sequence encodes an interferon, such as Interferon alpha 2b. (see, e.g. U.S. Patent No.: 6,835,557, herein incorporated by refrence in its entirety).

In other embodiments, the heterologous nucleotide sequence encodes an antibody. In yet other embodiments, the heterologous nucleotide sequence encodes a chimeric or fusion protein.

In certain embodiments, the heterologous nucleotide sequence encodes an antigenic protein, a polypeptide or peptide of a virus belonging to a different species, subgroup or variant of adenovirus other than the species, subgroup or variant from which the recombinant adenovirus vector is derived. In certain embodiments, the heterologous nucleotide sequence encodes an antigenic protein, polypeptide or peptide obtained and/or derived from a pathogenic microorganism.

In yet another embodiment of the invention, the heterologous nucleotide sequence is a cancer therapeutic gene. Such genes include those that enhance the antitumor activity of lymphocytes, genes whose expression product enhances the immunogenicity of tumor cells, tumor suppressor genes, toxin genes, suicide genes, multiple-drug resistance genes, antisense sequences, and the like. Thus, for example, the adenoviral vector of this invention can contain a foreign gene for the expression of a protein effective in regulating the cell cycle, such as p53, Rb, or mitosin, or in inducing cell death, such as the conditional suicide gene thymidine kinase.

As used herein the terms, "rAd production cell", "producer cell", and "packaging cell" are synonyms and mean a cell able to propagate recombinant adenoviruses by supplying a product required for efficient viral growth. A variety of mammalian cell lines are publicly available for the culture of recombinant adenoviruses. For example, the 293 cell line (Graham & Smiley, J. Gen Virol. 36:59-72 (1977)) has been engineered to complement the deficientcies of E1 function.

The rAd production cells or cell lines may be propagated using standard cell culture techniques (see *e.g*., R.I. Freshney, Culture of Animal Cells-A Manual of Basic Techniques, Second Edition, Wiley-Liss, Inc. New York, N.Y., 1987).

Cells may grow in serum-containing or serum-free conditions. The suspension culture may be shaken, rocked, agitated, rolled or stirred to maintain the cells in suspension.

"A549" is a lung carcinoma cell line which is commonly known in the art. In one embodiment, the A549 cell is ATCC strain CCL-185.

The recombinant adenovirus production cells or production cell line may be propagated or grown by any method known in the art for mammalian cell culture. Propagation may be done by a single step or a multiple step procedure. For example, in a single step propagation procedure, the production cells are removed from storage and inoculated directly to a culture vessel where production of virus is going to take place. In a multiple step propagation procedure, the production cells are removed from storage and propagated through a number of culture vessels of gradually increasing size until reaching the final culture vessel where the production is going to take place. During the propagation steps, the cells are grown under conditions that are optimized for growth. Culture conditions, such as temperature, pH, dissolved oxygen level and the like are those known to be optimal for the particular cell line and will be apparent to the skilled person or artisan within this field (see *e.g*., Animal Cell culture: A Practical Approach 2nd edition, Rickwood, D. and Hames, B.D. eds., Oxford University Press, New York (1992)).

The rAd production cells or rAd production cell lines may be grown and the rAd production cells or rAd production cells producing virus may be cultured in any suitable vessel which is known in the art. For example, cells may be grown and the infected cells may be cultured in a biogenerator or a bioreactor. Generally, "biogenerator" or "bioreactor" means a culture tank, generally made of stainless steel, or glass, with a volume of 0.5 liter or greater, comprising an agitation system, a device for injecting a stream of CO₂ gas and an oxygenation device. Typically, it is equipped with probes measuring the internal parameters of the biogenerator, such as the pH, the dissolved oxygen, the temperature, the tank pressure or certain physicochemical parameters of the culture (for instance the consumption of glucose or of glutamine or the production of lactate and ammonium ions). The pH, oxygen, and temperature probes are connected to a bioprocessor which permanently regulates these parameters. In another embodiment, the vessel is a WAVE Bioreactor (WAVE Biotech, Bridgewater, NJ, U.S.A.).

Cell density in the culture may be determined by any method known in the art. For example, cell density may be determined microscopically (*e.g*., hemacytometer) or by an electronic cell counting device (*e.g*., COULTER COUNTER; AccuSizer 780/SPOS Single Particle Optical Sizer).

The term "infecting" means exposing the recombinant adenovirus to the cells or cell line under conditions so as to facilitate the infection of the cell with the recombinant adenovirus. In cells which have been infected by multiple copies of a given virus, the activities necessary for viral replication and virion packaging are cooperative. Thus, it is preferred that conditions be adjusted such that there is a significant probability that the cells are multiply infected with the virus. An example of a condition which enhances the production of virus in the cell is an increased virus concentration in the infection phase. However, it is possible that the total number of viral infections per cell can be overdone, resulting in toxic effects to the cell. Consequently, one should strive to maintain the infections in the virus concentration in the range of 10⁶ to 10¹⁰, preferably about 10⁹, virions per ml. Chemical agents may also be employed to increase the infectivity of the cell line. For example, the present invention provides a method to increase the infectivity of cell lines for viral infectivity by the inclusion of a calpain inhibitor. Examples of calpain inhibitors useful in the practice of the present invention include calpain inhibitor I (also known as N-acetyl-leucyl-leucyl-norleucinal, commercially available from Boehringer Mannheim). Calpain inhibitor I has been observed to increase the infectivity of cell lines to recombinant adenovirus.

The term "culturing under conditions to permit replication of the viral genome" means maintaining the conditions for the infected cell so as to permit the virus to propagate in the cell. It is desirable to control conditions so as to maximize the number of viral particles produced by each cell. Consequently, it will be necessary to monitor and control reaction conditions such as, for example, temperature, dissolved oxygen and pH level. Commercially available bioreactors such as the CelliGen Plus Bioreactor (commercially available from New Brunswick Scientific, Inc. 44 Talmadge Road, Edison, NJ) have provisions for monitoring and maintaining such parameters. Optimization of infection and culture conditions will vary somewhat, however, conditions for the efficient replication and production of virus may be achieved by those of skill in the art taking into considerations the known properties of the producer cell line, properties of the virus, and the type of bioreactor.

Virus, such as adenovirus, may be produced in the cells. Virus may be produced by culturing the cells; optionally adding fresh growth medium to the cells; inoculating the cells with the virus; incubating the inoculated cells (for any period of time); optionally adding fresh growth medium to the inoculated cells; and optionally harvesting the virus from the cells and the medium. Typically, when the concentration of viral particles, as determined by conventional methods, such as high performance liquid chromatography using a Resource Q column, as described in Shabram, et al. Human Gene Therapy 8:453-465 (1997), begins to plateau, the harvest is performed.

Fresh growth medium may be provided to the inoculated cells at any point. For example, the fresh medium may be added by perfusion. Medium exchange may significantly increase virus production in the cells. In one embodiment of the invention, the medium of cells is subject to two consecutive exchanges - one upon infection and another one day post-infection.

The cells used to produce the virus may be derived from a cell line frozen under serum-free medium conditions or from a cell line frozen under serum-containing medium conditions (*e.g*., from a frozen cell bank).

Suitable methods for identifying the presence of the virus in the culture, *i.e*., demonstrating the presence of viral proteins in the culture, include immunofluorescence tests, which may use a monoclonal antibody against one of the viral proteins or polyclonal antibodies (Von Bülow et al., in Diseases of Poultry, 10th edition, Iowa State University Press), polymerase chain reaction (PCR) or nested PCR (Soiné et al., Avian Diseases 37:467-476 (1993)), ELISA (Von Bülow et al., in Diseases of Poultry, 10th edition, Iowa State University Press)), hexon expression analyzed by flow cytometry (Musco et al. Cytometry 33:290-296 (1998), virus neutralization, or any of the common histochemical methods of identifying specific viral proteins.

Titrating the quantity of the adenovirus in the culture may be performed by techniques known in the art. In a particular embodiment, the concentration of viral particles is determined by the Resource Q assay as described by Shabram, et al. Human Gene Therapy 8:453-465 (1997). As used herein, the term "lysis" refers to the rupture of the virus-containing cells. Lysis may be achieved by a variety of means well known in the art. For example, mammalian cells may be lysed under low pressure (100-200 psi differential pressure) conditions, by homogenization, by microfluidization, or by conventional freeze-thaw methods. Exogenous free DNA/RNA may be removed by degradation with DNAse/RNAse.

The adenovirus-containing cells may be frozen. Adenovirus may be harvested from the virus-containing cells and the medium. In one embodiment, the adenovirus is harvested from both the virus-containing cells and the medium simultaneously. In a particular embodiment, the adenovirus producing cells and medium are subjected to cross-flow microfiltration, as described, for example, in U.S. Patent Number 6,146,891, under conditions to both simultaneously lyse virus-containing cells and clarify the medium of cell debris which would otherwise interfere with virus purification.

The term "harvesting" means the collection of the cells containing the adenovirus from the media and may include collection of the adenovirus from the media. This may be achieved by conventional methods such as differential centrifugation or chromatographic means. At this stage, the harvested cells may be stored frozen or further processed by lysis and purification to isolate the virus. Exogenase free DNA/RNA may be removed by degradation with DNAse/RNAse, such as BENZONASE (American International Chemicals, Inc.).

The virus harvest may be further processed to concentrate the virus by methods such as ultrafiltration or tangential flow filtration as described in U.S. Patent Numbers 6,146,891 and 6,544,769.

The term "recovering" means the isolation of a substantially pure population of recombinant virus particles from the lysed producer cells and optionally from the supernatant medium. Viral particles produced in the cell cultures of the present invention may be isolated and purified by any method which is commonly known in the art. Conventional purification techniques such as chromatographic or differential density gradient centrifugation methods may be employed. For example, the viral particles may be purified by cesium chloride gradient purification, column or batch chromatography, diethylaminoethyl (DEAE) chromatography (Haruna et al. Virology 13: 264-267 (1961); Klemperer et al., Virology 9: 536-545 (1959); Philipson et al., Virology 10: 459-465 (1960)), hydroxyapatite chromatography (U.S. Patent Application Publication Number US2002/0064860) and chromatography using other resins such as homogeneous cross-linked polysaccharides, which include soft gels (*e.g*., agarose), macroporous polymers "throughpores", "tentacular" sorbents, which have tentacles that were designed for faster interactions with proteins (*e.g*., fractogel) and materials based on a soft gel in a rigid shell, which exploit the high capacity of soft gels and the rigidity of composite materials (*e*.*g*., Ceramic HyperD® F) (Boschetti, Chromatogr. 658:207 (1994); Rodriguez, J. Chromatogr. 699:47-61 (1997)). In the preferred practice of the invention, the virus is purified by column chromatography in substantial accordance with the process of Huyghe et al., Human Gene Therapy 6:1403-1416 (1995) as described in Shabram *et al*., United States Patent 5,837,520 issued November 17, 1998, and United States Patent 6,261,823.

### EXAMPLES

Generally following the protocols described in Molecular Therapy, 2005 Aug;12(2):254-63*; we evaluated the cardiovascular response in mice administered either Peglyated or non-Pegylated recombinant adenovirus. Both EKG and heart rate were evaluated. The recombinant adenovirus was Pegylated with* SPA-PEG5K linker. The results are based on an n of 4 and 5 in the various experiments.

It was observed that PEGylation of rAd-vectors inhibited the hemodynamic responses in half the treated mice in three separate experiments. Inhibition of hemodynamic cardiovascular responses in normal mice could be achieved using rAd-vectors with about 600-800 PEG molecules per virion.

The effects of PEGylation on systemic transduction *in vivo* were assessed in mice infused by intravenous route with 1 x 10¹¹ particles of unmodified or PEGylated rAd-vector 3-days later. Biodistribution was assessed by quantitative real time PCR and RT-PCR in liver, spleen, adrenal, lung, small & large intestine, kidney and heart tissues to assess relative transduction capacity of the various forms. Surprisingly, transduction efficacy in the various organs was relatively equivalent for the unmodified and the PEG-rAd vectors. Biodistribution of the transgene specific DNA was essentially equivalent in all organs examined and compared to the house keeping GAPDH gene expression. These results indicated that transduction efficacy was similar to non-PEGylated rAd-vector using rAd-PEG with 600-800 PEG-molecules / virion.

Also tested was the capacity of rAd-vector PEGylation to mitigate adverse events in a model of anaphylactoid responses. This model provided us with the capacity to examine the rAd-PEG vectors with varying degrees of PEGylation on the surface resulted in mitigation of anaphylactoid responses and to correlate the degree of PEGylation (i.e., number of PEG-molecules covalently bonded per virion particle) required to inhibit exacerbated responses associated with chronic inflammation. PEGylation of rAd-vectors with greater than 1250 PEG-molecules and up to 1600 PEG-molecules per virion (8% SPA-PEG during PEGylation reaction) resulted in inhibition of anaphylactoid responses.

## Claims

1. Use of a PEGylated recombinant adenovirus virion **in the manufacture of a medicament** for mitigating adverse effects associated with systemic administration of a recombinant adenovirus for gene therapy, wherein the degree of PEGylation of the adenovirus virion is between about 400 PEG molecules per virion and about 1250 PEG molecules per virion and the adverse effect is a cardiovascular adverse effect, an anaphylactoid response, coagulopathy or inflammation.

2. The use-of claim 1, wherein the adverse effect is a cardiovascular adverse effect

3. The use of claim 1, wherein the adverse effect is an anaphylactoid response.

4. The use of claim 1, wherein the adverse effect is coagulopathy.

5. The use of claim 1, wherein the adverse effect is inflammation.

6. The use of claim 1, wherein the transduction efficiency of the PEGylated recombinant adenovirus is equal to or greater then a transduction efficiency of a non-PEGylated recombinant adenovirus.

7. The use of claim 1, wherein the PEG linker is PEG-SPA linker with a PEG molecular weight of 5kD.

8. The use of claim 1, wherein the degree of PEGylation of the adenovirus virion is between about 600 PEG molecules per virion and about 1000 PEG molecules per virion.

## Patentansprüche

1. Verwendung eines PEGylierten rekombinanten Adenovirus-Virions in der Herstellung eines Medikamentes für das Lindern der nachteiligen Wirkungen, die mit der systemischen Verabreichung eines rekombinanten Adenovirus für die Gentherapie verbunden sind, wobei der Grad der PEGylierung des Adenovirus-Virions zwischen ungefähr 400 PEG Molekülen je Virion und ungefähr 1250 PEG Molekülen je Virion liegt und die nachteilige Wirkung eine kardiovaskulär nachteilige Wirkung, eine anaphylaktoide Antwort, Koagulopathie oder eine Entzündung ist.

2. Verwendung gemäss Anspruch 1, wobei die nachteilige Wirkung eine kardiovaskulär nachteilige Wirkung ist.

3. Verwendung gemäss Anspruch 1, wobei die nachteilige Wirkung eine anaphylaktoide Antwort ist.

4. Verwendung gemäss Anspruch 1, wobei die nachteilige Wirkung Koagulopathie ist.

5. Verwendung gemäss Anspruch 1, wobei die nachteilige Wirkung eine Entzündung ist.

6. Verwendung gemäss Anspruch 1, wobei die Transduktionseffizienz des PEGylierten rekombinanten Adenovirus gleich oder grösser als eine Transduktionseffizienz eines nicht PEGylierten rekombinanten Adenovirus ist.

7. Verwendung gemäss Anspruch 1, wobei der PEG-Verbinder ein PEG-SPA-Verbinder mit einem PEG-Molekulargewicht von 5 kD ist.

8. Verwendung gemäss Anspruch 1, wobei der Grad der PEGylierung des Adenovirus Virions zwischen ungefähr 600 PEG Molekülen je Virion und ungefähr 1000 PEG Molekülen je Virion liegt.

## Revendications

1. Utilisation d'un virion d'adénovirus recombinant PEGylé dans la fabrication d'un médicament pour atténuer les effets indésirables associés à l'administration systémique d'un adénovirus recombinant pour une thérapie génique, dans laquelle le degré de PEGylation du virion d'adenovirus est comprise entre environ 400 molécules de PEG par virion et environ 1250 molécules de PEG par virion et l'effet indésirable est un effet indésirable d'origine cardiovasculaire, une réponse anaphylactoïde, une coagulopathie ou une inflammation.

2. L'utilisation-selon la revendication 1, dans laquelle l'effet indésirable consiste en un effet indésirable d'origine cardiovasculaire.

3. L'utilisation selon la revendication 1, dans laquelle l'effet indésirable est une réponse anaphylactoïde.

4. L'utilisation selon la revendication 1, dans laquelle l'effet indésirable est une coagulopathie.

5. L'utilisation selon la revendication 1, dans laquelle l'effet indésirable est une inflammation.

6. L'utilisation selon la revendication 1, dans laquelle l'efficacité de la transduction d'adénovirus recombinants PEGylés est égale ou supérieure à l'efficacité de la transduction d'un adénovirus recombinant non-PEGylé.

7. L'utilisation selon la revendication 1, dans laquelle le lieur PEG est un lieur PEG-SPA présentant un poids moléculaire du PEG de 5kD.

8. L'utilisation selon la revendication 1, dans laquelle le degré de PEGylation du virion d'adénovirus est comprise entre environ 600 molécules de PEG par virion et environ 1000 molécules PEG par virion.
